# EUROPÄISCHE PATENTANMELDUNG

(11) **EP 4 772 150 A1**
(43) Veröffentlichungstag der Anmeldung: **08.07.2026**
(21) Anmeldenummer: 26150141.5
(22) Anmeldetag: 05.01.2026
(51) Int. Cl.: A61F 9/00, A61M 37/00, A61K 9/00

(54) **INJEKTOR FÜR EIN IMPLANTAT**

(30) Priorität: 07.01.2025 DE 102025000049
(71) Anmelder: IMSTec GmbH, 55270 Klein-Winternheim (DE)
(72) Erfinder: Mähringer-Kunz, Edgar, 55424 Münster-Sarmsheim (DE)
(74) Vertreter: Gottschald Patentanwälte Partnerschaft mbB

(57) **Zusammenfassung**

Die Erfindung betrifft einen Injektor für ein Implantat (2), wobei das Implantat (2) in dem Injektor (1) gelagert ist, wobei der Injektor (1) eine Injektionsöffnung (3) zum Abgeben des Implantats (2) in ein Organ, insbesondere ein Auge, einen Lagerabschnitt (4), in dem das Implantat (2) gelagert ist, und einen Vorschubmechanismus (5) zum Ausstoßen des Implantats (2) von dem Lagerabschnitt (4) durch die Injektionsöffnung (3) aufweist. Es wird vorgeschlagen, dass das Implantat (2) mittels eines das Implantat (2) kontaktierenden Halteelements (7) im Lagerabschnitt (4) festgelegt ist.

## Beschreibung

Die vorliegende Erfindung betrifft einen Injektor für ein Implantat gemäß dem Oberbegriff von Anspruch 1, eine Injektoranordnung gemäß dem Oberbegriff von Anspruch 9 sowie ein Verfahren zur Herstellung eines Injektors gemäß dem Oberbegriff von Anspruch 10.

Im Fokus stehen vorliegend ophthalmologische, pharmazeutisch wirksame Implantate zur Behandlung von Krankheiten des menschlichen Auges. Solche Implantate können mittels eines speziellen Injektors, der oft als Spritze mit einer nadelförmigen Spitze ausgebildet ist, in das Auge eines Patienten appliziert werden. Im Auge wird das Implantat über die Zeit biologisch abgebaut und setzt währenddessen einen Wirkstoff frei. Das Implantat kann dafür beispielsweise eine Polymermatrix aufweisen, die den Wirkstoff trägt. Der Injektor kann als Einweginjektor ausgestaltet sein und bereits mit dem Implantat geliefert werden, sodass die Applikation des Implantats in das Auge durch eine Entnahme des Injektors aus einer sterilisierten Verpackung und anschließendes Stechen des Injektors in das Auge besonders effizient und wenig fehleranfällig durchführbar ist. Genauso kann jedoch ein Teil des Injektors wiederverwendbar und sterilisierbar sein. Um das Implantat auszustoßen, kann der Injektor einen, in der Regel manuell betätigbaren, Vorschubmechanismus aufweisen, mit dem das Implantat durch eine Injektionsöffnung geschoben werden kann.

Ein Problem bei solchen Injektoren ist, sicherzustellen, dass das Implantat während des Transports und bei der Handhabung des Injektors seine Position in einem Lagerabschnitt beibehält und nicht in eine ungünstige Lage, in der das Vorschieben erschwert oder verhindert wird, rutscht, aus dem Injektor fällt oder beschädigt wird.

Das obige Problem wird durch die Merkmale des kennzeichnenden Teils von Anspruch 1 gelöst.

Im Fokus steht die grundsätzliche Überlegung, dass der Injektor mit einem Halteelement versehen werden kann, das das Implantat im Lagerabschnitt festlegt.

Im Einzelnen wird vorgeschlagen, dass das Implantat mittels eines das Implantat kontaktierenden Halteelements im Lagerabschnitt festgelegt ist. Der Begriff "festgelegt" meint dabei, dass das Halteelement das Implantat ortsfest hält.

Besonders bevorzugt ist das Halteelement biokompatibel. Aufgrund der Biokompatibilität des Halteelements bestehen keine Bedenken, wenn das Halteelement nicht vollständig im Injektor verbleibt und somit mit dem Implantat implantiert wird. Entsprechend kann bei einer Ausgestaltung gemäß Anspruch 2 vorgesehen sein, dass das Halteelement beim Ausstoßen des Implantats zumindest teilweise zerstört wird und/oder zumindest teilweise am Implantat haften bleibt. Besonders problemlos ist ein Einbringen eines Teils des Halteelements in einen Patienten, wenn das Halteelement aus einem Material besteht, aus dem auch das Implantat, zumindest teilweise, besteht. Anspruch 3 gibt entsprechend bevorzugte Materialien für Implantat und Injektor an.

Bei einer Ausgestaltung gemäß Anspruch 4 kann vorgesehen sein, dass das Halteelement am Implantat und am Lagerabschnitt haftet. Unter Haftung kann eine ausreichend starke Haftreibung verstanden werden, die bei üblichen Beschleunigungen des Injektors während des Transports und dergleichen aufgrund der geringen Masse des Implantats ausreichend ist. Bevorzugt umfasst die Haftung jedoch einen stoffschlüssigen Anteil. Es lässt sich mithin sagen, dass das Implantat über das Halteelement an dem Lagerabschnitt festgeklebt sein kann. Das Halteelement kann demnach ein Tropfen eines klebenden Materials oder dergleichen sein.

Anspruch 5 betrifft Ausgestaltungen des Injektors und speziell eines vorderen Abschnitts, der den Lagerabschnitt und die Injektionsöffnung aufweist. Das Halteelement kann durch eine Öffnung eingebracht werden, die separat von der Injektionsöffnung ist, wodurch beispielsweise erreicht werden kann, dass das Halteelement nicht im Weg des Implantats angeordnet ist, damit das Implantat nicht mit dem Halteelement kollidiert, sodass das Ausstoßen des Implantats reproduzierbar erfolgt. Besonders einfach kann eine hintere Öffnung des vorderen Abschnitts genutzt werden, die auch für den Vorschubmechanismus Verwendung findet.

Produktionstechnisch interessant ist auch die Möglichkeit, zum Einbringen des Halteelements eine seitliche Öffnung des vorderen Abschnitts zu verwenden. So kann die Komplexität der Fertigungsschritte des Injektors reduziert werden und zusätzlich kann das Halteelement teilweise außerhalb eines Bewegungsweges des Implantats und/oder des Vorschubmechanismus angeordnet sein.

Der vordere Abschnitt kann als separates Bauteil ausgefertigt sein, wodurch das Implantat einfach von hinten eingebracht werden kann, bevor der Injektor zusammengesetzt wird (Anspruch 6).

Das Halteelement kann konsequenterweise an einem hinteren Abschnitt des Implantats angeordnet sein (Anspruch 7).

Anspruch 8 betrifft weitere bevorzugte konstruktive Merkmale des Vorschubmechanismus.

Nach einer weiteren Lehre gemäß Anspruch 9, der eigenständige Bedeutung zukommt, wird eine Injektoranordnung beansprucht.

Dabei wird vorgeschlagen, dass der Injektor oder der vordere Abschnitt steril verpackt ist.

Auf alle Ausführungen zu dem vorschlagsgemäßen Injektor darf verwiesen werden.

Nach einer weiteren Lehre gemäß Anspruch 10, der ebenfalls eigenständige Bedeutung zukommt, wird ein Verfahren zur Herstellung eines Injektors beansprucht.

Dabei wird vorgeschlagen, dass das Implantat und das Halteelement in den Lagerabschnitt eingebracht werden.

Auf alle Ausführungen zu dem vorschlagsgemäßen Injektor und der vorschlagsgemäßen Injektoranordnung darf verwiesen werden.

In verschiedenen Ausgestaltungen gemäß Anspruch 11 ist beschrieben, wie das Halteelement und das Implantat in den Lagerabschnitt eingebracht werden können. Zum Einbringen des Halteelements und/oder des Implantats kann gemäß Anspruch 12 die bereits erwähnte weitere Öffnung verwendet werden.

Beim Einbringen kann das Halteelement einen flüssigen Zustand aufweisen (Anspruch 13) und/oder nach dem Einbringen aktiv ausgehärtet werden (Anspruch 14).

Bevorzugt handelt es sich bei dem Injektor um einen Injektor, der herstellerseitig bestückt und anschließend sterilisiert wird (Einweginjektor). Alternativ kann ein Teil des Injektors herstellerseitig bestückt und sterilisiert werden.

Im Folgenden wird die Erfindung anhand einer lediglich Ausführungsbeispiele darstellenden Zeichnung näher erläutert. In der Zeichnung zeigt
- Fig. 1: einen vorschlagsgemäßen Injektor mit ausgestoßenem Implantat,
- Fig. 2: Schnittansichten des Injektors in unterschiedlichen Zuständen,
- Fig. 3: mögliche Wege, das Implantat in den Injektor einzubringen und dort festzulegen,
- Fig. 4: weitere Wege, das Implantat in den Injektor einzubringen und dort festzulegen und
- Fig. 5: ein federbasiertes Halteelement.

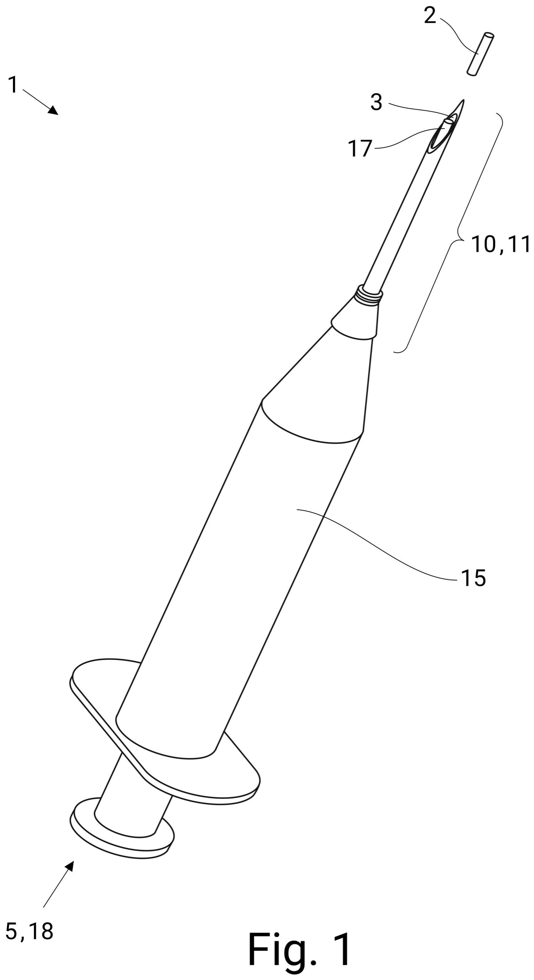

Die in den Figuren dargestellten und insoweit bevorzugten Ausführungsbeispiele betreffen einen Injektor 1 für ein Implantat 2. Fig. 1 zeigt eine Außenansicht eines solchen Injektors 1, bei dem das Implantat 2 bereits außerhalb des Injektors 1 dargestellt ist. Bei dem Implantat 2 handelt es sich hier und vorzugsweise um ein ophthalmologisches Implantat 2, das vorzugsweise pharmazeutisch wirksam ist. Beispielsweise kann es sich bei dem Implantat 2 um ein Medikament handeln, das ins Auge, beispielsweise den Glaskörper, injiziert wird. Im Auge wird das Implantat 2 dann, insbesondere vollständig, abgebaut und gibt währenddessen einen Wirkstoff frei. Das Implantat 2 ist demnach vorzugsweise vollständig biologisch abbaubar. Dieser Abbau kann einige Monate dauern, wodurch auch chronische, rezidivierende oder allgemein hartnäckige Erkrankungen therapiert werden können. Verwendung finden solche Implantate 2 beispielsweise bei altersbedingter Makuladegeneration, einem diabetischen Makulaödem oder Infektionen des hinteren Augensegments.

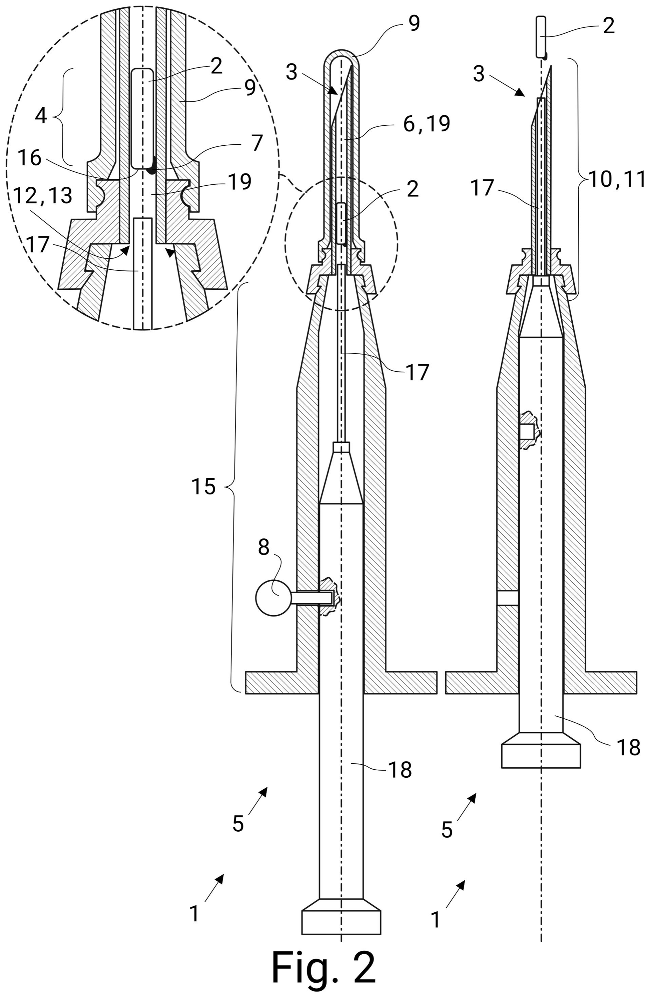

Mit Blick auf den Injektor 1 und beispielsweise Fig. 2 ist erkennbar, dass das Implantat 2 in dem Injektor 1 gelagert ist. Vorzugsweise handelt es sich bei dem Injektor 1 um einen Einweginjektor, womit das Implantat 2 herstellerseitig in den Injektor 1 eingebracht wurde. Der Injektor 1 weist eine Injektionsöffnung 3 zum Abgeben des Implantats 2 in ein Organ, insbesondere ein Auge, einen Lagerabschnitt 4, in dem das Implantat 2 gelagert ist, und einen Vorschubmechanismus 5 zum Ausstoßen des Implantats 2 von dem Lagerabschnitt 4 durch die Injektionsöffnung 3 auf. Insgesamt kann der Injektor 1 spritzenartig ausgestaltet sein, sodass die Injektionsöffnung 3 ohne vorherige Inzision in das Organ, hier das Auge, gestochen werden kann. Nach einem solchen Einstich wird das Implantat 2 durch den Vorschubmechanismus 5 aus der, dann im Organ angeordneten, Injektionsöffnung 3 ausgestoßen. Je nach Ausgestaltung kann vorgesehen sein, dass der Vorschubmechanismus 5 bereits vor dem Einstich in das Organ betätigt wird, um das Implantat 2 vom Lagerabschnitt 4 vorzuschieben und vorzugsweise nah an der Injektionsöffnung 3 anzuordnen.

Der Lagerabschnitt 4 ist hier und vorzugsweise von der Injektionsöffnung 3 beabstandet und kann mit der Injektionsöffnung 3 über einen vorderen Kanalabschnitt 6 verbunden sein. Das Implantat 2 wird dann bei der Verwendung des Injektors 1 durch den Vorschubmechanismus 5 von dem Lagerabschnitt 4 durch den vorderen Kanalabschnitt 6 befördert, insbesondere geschoben, und anschließend aus der Injektionsöffnung 3 ausgestoßen.

Vorgeschlagen wird nun, dass das Implantat 2 mittels eines das Implantat 2 kontaktierenden Halteelements 7 im Lagerabschnitt 4 festgelegt ist. Vorzugsweise kontaktiert das Halteelement 7 das Implantat 2 aus radialer Richtung. Die radiale Richtung ist dabei orthogonal zu einer Längsrichtung des Injektors 1, entlang der das Implantat 2 bewegt wird, orientiert.

Fig. 2 zeigt links einen Injektor 1 im Transportzustand und rechts einen Injektor 1 mit ausgestoßenem Implantat 2. Der Injektor 1 weist hier und vorzugsweise eine Transportsicherung 8 auf, die in einem aktiven Zustand eine Bewegung des Vorschubmechanismus 5 verhindert. Außerdem weist der Injektor 1 hier und vorzugsweise eine Schutzkappe 9 auf, die die Injektionsöffnung 3 überdeckt.

Bevorzugt ist es so, dass das Implantat 2 biokompatibel ist. Das Halteelement 7 ist hier und vorzugsweise separat von dem Injektor 1 ausgestaltet. Der Begriff "Halteelement" ist dabei weit zu verstehen, insbesondere kann es sich bei dem Halteelement 7 um einen Tropfen eines Materials halten, das stoffschlüssig das Implantat 2 mit dem Lagerabschnitt 4 verbindet und somit eine ähnliche Funktion wie ein Klebstoff übernimmt. Es wird allerdings angemerkt, dass das Ausstoßen des Implantats 2 nicht wesentlich erschwert werden sollte. Dennoch verhindert das Halteelement 7 zumindest für übliche, während des Transports und/oder der Handhabung des Injektors 1 auftretende Beschleunigungen in Kombination mit der geringen Masse des Implantats 2, dass das Implantat 2 den Lagerabschnitt 4 verlässt.

Aufgrund der Biokompatibilität des Halteelements 7 ist denkbar, dass das Halteelement 7 beim Ausstoßen des Implantats 2 zumindest teilweise zerstört wird. Teile des Halteelements 7, die mit in das Organ injiziert werden, sind so unkritisch. Diese Ausgestaltung zeigt, dass aufgrund der Biokompatibilität eine einfache Festlegung des Implantats 2 im Lagerabschnitt 4 möglich ist, da nicht sichergestellt werden muss, dass das Halteelement 7 im Injektor 1 verbleibt.

Denkbar ist somit zusätzlich oder alternativ, dass das Halteelement 7 beim Ausstoßen zumindest teilweise am Implantat 2 haften bleibt.

Besonders interessant ist die Möglichkeit, dass das Halteelement 7, insbesondere vollständig, aus einem Material besteht, aus dem das Implantat 2, zumindest teilweise, ebenfalls besteht. So bestehen auch keine Bedenken hinsichtlich der Verträglichkeit oder dergleichen des Halteelements 7. Möglich ist allerdings grundsätzlich, dass das Halteelement 7 biologisch inert und/oder biologisch abbaubar ist, auch wenn andere Materialien als beim Implantat 2 genutzt werden.

Weiter ist hier und vorzugsweise vorgesehen, dass das Halteelement 7, zumindest teilweise, aus einem Polymer besteht, vorzugsweise, dass das Polymer Polycaprolacton (PCL) oder Polylactid (PLA) oder Polyglycolsäure (PGA) oder Polylactid-co-Glycolid (PLGA) ist. Eines dieser Polymere kann als Bestandteil des Implantats 2 eine Matrix ausbilden, die ein Arzneimittel trägt.

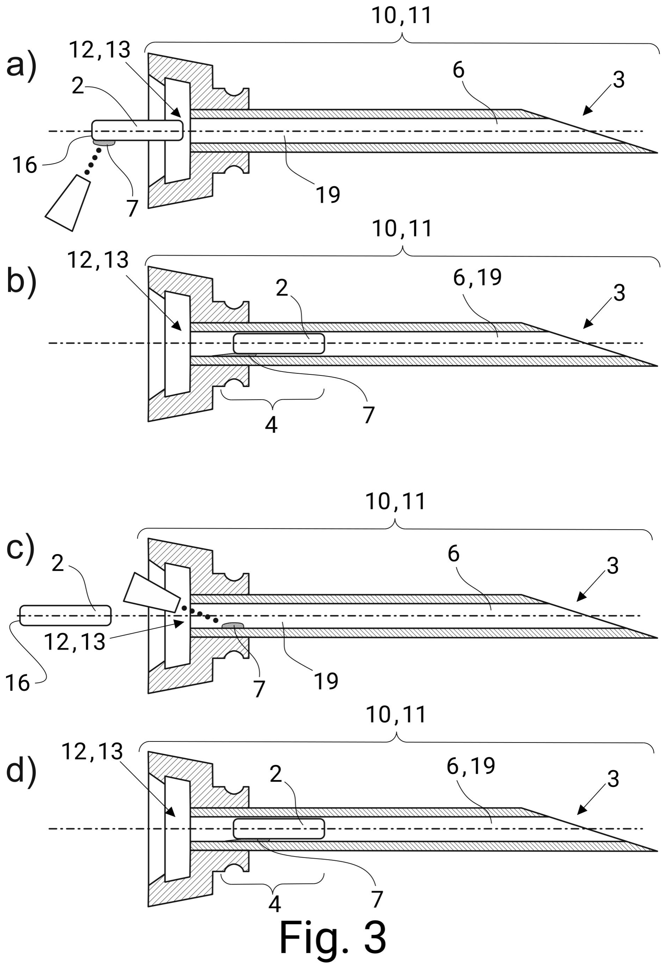

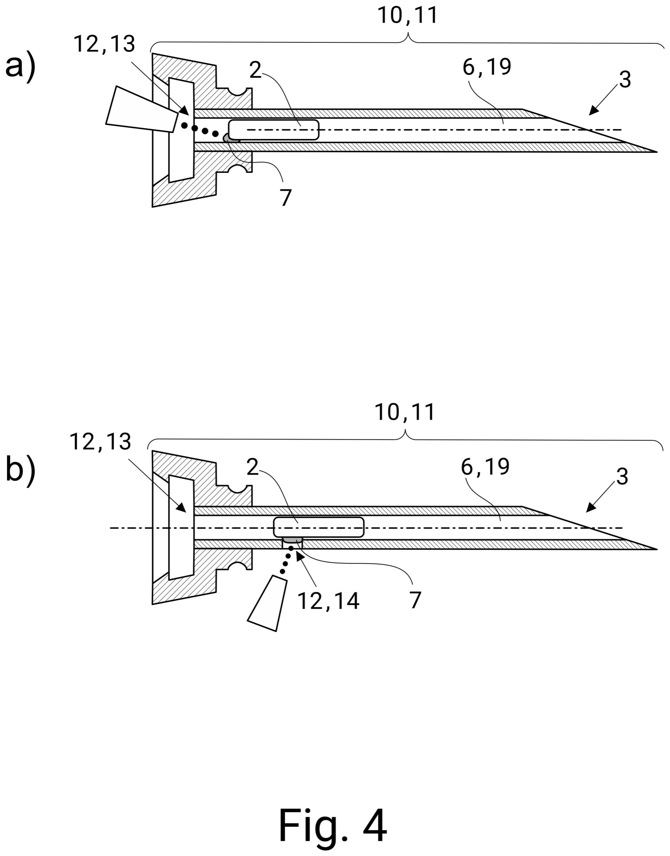

Es ist hier und vorzugsweise vorgesehen, dass das Halteelement 7 am Implantat 2 und am Lagerabschnitt 4 haftet. Vorzugsweise ist vorgesehen, dass das Halteelement 7 am Implantat 2 und/oder am Lagerabschnitt 4 stoffschlüssig haftet. In der Summe kann das Halteelement 7 daher wie erwähnt das Implantat 2 an den Lagerabschnitt 4 kleben, wobei wie erwähnt die Haftkraft zwischen Halteeffekt und einfachem Ausstoßen des Implantats 2 abgewogen werden muss. Die Figuren 3 und 4 zeigen mehrere Varianten, wie Halteelement 7 und Implantat 2 in den Injektor 1 eingebracht werden können. Diese werden nachfolgend noch erläutert. Interessant ist an dieser Stelle schonmal die Fig. 4 b), die zeigt, dass das Halteelement 7 durchaus auch, zumindest teilweise, formschlüssig am Lagerabschnitt 4 festgelegt sein kann. In diesem konkreten Fall ist eine Kombination aus Form- und Stoffschluss vorgesehen.

Genauso ist denkbar, dass zwischen dem Halteelement 7 und dem Implantat 2 und/oder zwischen dem Halteelement 7 und dem Lagerabschnitt 4 ein Reibschluss vorliegt, insbesondere ohne Formschluss und/oder ohne Stoffschluss. Es versteht sich außerdem, dass vorzugsweise das Halteelement 7 im Lagerabschnitt 4 angeordnet ist (Figuren 2, 3, 4 a) und 5).

Bezüglich der Ausgestaltung des Injektors 1 kann vorgesehen sein, dass der Injektor 1 einen vorderen Abschnitt 10 aufweist, der insbesondere als hohle Nadelspitze 11 ausgestaltet ist, und der den Lagerabschnitt 4 und die Injektionsöffnung 3 aufweist.

Der vordere Abschnitt 10 kann zusätzlich zur Injektionsöffnung 3 eine weitere Öffnung 12 aufweisen, durch die das Halteelement 7 eingebracht wurde. Vorzugsweise ist die weitere Öffnung 12 eine hintere Öffnung 13 des vorderen Abschnitts 10, durch die der Vorschubmechanismus 5 hindurch bewegt wird (Fig. 3, Fig. 4a)).

Alternativ kann die weitere Öffnung 12 eine seitliche Öffnung 14 sein, die vorzugsweise nur dem Einbringen des Halteelements 7 dient und die weiter vorzugsweise von dem Halteelement 7 verschlossen wird (Fig. 4b)). Allgemein ist das Vorsehen einer seitlichen Öffnung 14 im vorderen Abschnitt 10 des Injektors 1 interessant, da das Halteelement 7 so seitlich des Implantats 2 appliziert werden kann. Durch die seitliche Öffnung 14 ist das Implantat 2 in der Regel besser zugänglich und ein Teil des Halteelements 7 kann in der seitlichen Öffnung 14 verbleiben. Der Vorschubmechanismus 5 kann dann besser an dem Halteelement 7 vorbeigeschoben werden. Die seitliche Öffnung 14 kann beispielsweise eine Bohrung in der Nadelspitze 11 sein. Die seitliche Öffnung 14 kann in einer hinteren Hälfte des vorderen Abschnitts 10 angeordnet sein. Die seitliche Öffnung 14 kann schräg, insbesondere orthogonal, zu einer Längserstreckung des vorderen Abschnitts 10 verlaufen.

Ein Blick auf die Figuren 3 und 4 zeigt, dass hier und vorzugsweise der vordere Abschnitt 10 als separates Bauteil ausgestaltet ist. Dadurch ist es besonders einfach möglich, das Implantat 2 durch die hintere Öffnung 13 in den vorderen Abschnitt 10 einzubringen und den Injektor 1 erst anschließend zusammenzubauen. Vorzugsweise ist es weiter so, dass der Injektor 1 einen Hauptabschnitt 15 aufweist, in dem der Vorschubmechanismus 5 verschiebbar geführt ist und an dem der vordere Abschnitt 10 befestigt ist. Die Befestigung ist in Fig. 2 als Vergrößerung dargestellt. Außerdem kann der Hauptabschnitt 15, wie bereits erwähnt, eine Transportsicherung 8 für den Vorschubmechanismus 5 aufweisen.

Der Vorschubmechanismus 5 und/oder der vordere Abschnitt 10 können aus Stahl gefertigt sein oder Stahl aufweisen. Der Hauptabschnitt 15 ist vorzugsweise spritzgegossen.

Aus den Figuren ergibt sich, dass das Halteelement 7 in einer hinteren, von der Injektionsöffnung 3 abgewandten, Hälfte, insbesondere einem hinteren Drittel, des Implantats 2 an dem Implantat 2 haftet. Dabei ist es vorzugsweise so, dass das Halteelement 7 an einem hinteren Ende 16 des Implantats 2 haftet.

Weiter kann vorgesehen sein, dass der Vorschubmechanismus 5 ein Vorschubelement 17 und ein Antriebselement 18 aufweist, dass das Antriebselement 18 manuell von außerhalb des Injektors 1 verstellbar ist und das Vorschubelement 17, insbesondere linear, vorschiebt, und, dass das Vorschubelement 17 von hinten in Eingriff mit dem Implantat 2 kommt und das Implantat 2 vorschiebt.

Der Injektor 1 kann insgesamt einen Kanal 19 aufweisen, der im vorderen Abschnitt 10 und im Hauptabschnitt 15 angeordnet sein kann. In dem Kanal 19 sind vorzugsweise ein Vorschubelement 17 des Vorschubmechanismus 5, das mit dem Implantat 2 in Eingriff kommt, und das Implantat 2 angeordnet. Der Kanal 19 mündet vorzugsweise in die Injektionsöffnung 3.

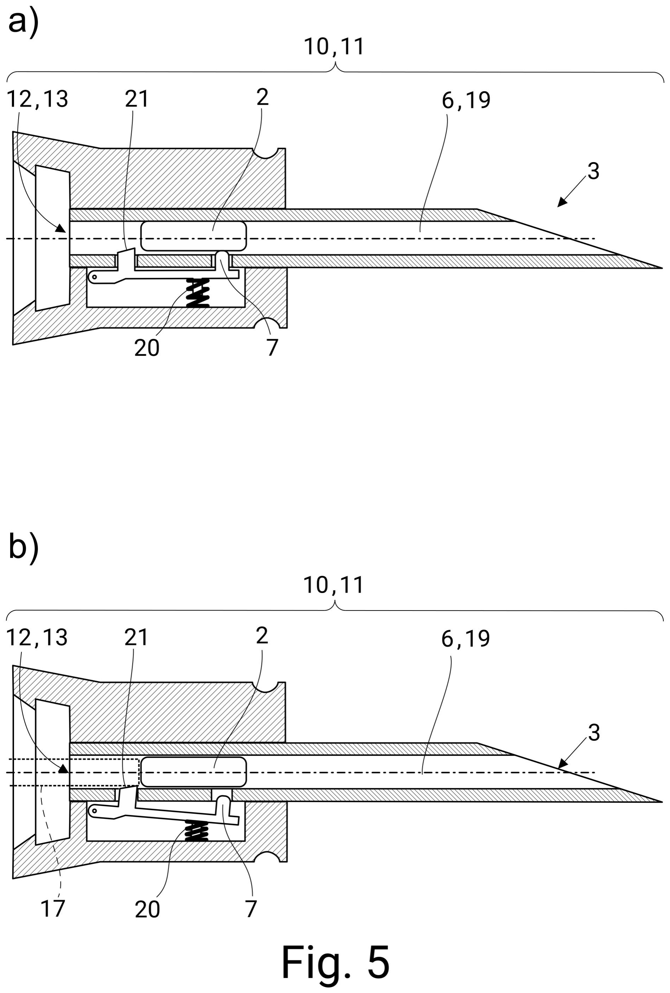

Fig. 5 zeigt eine Ausgestaltung, bei der das Halteelement 7 federbeaufschlagt ist. Allgemein kann also vorgesehen sein, dass das Halteelement 7 das Implantat 2 durch eine Federkraft im Lagerabschnitt 4 festlegt. Der Injektor 1 kann demnach eine Feder 20 aufweisen. Hier und vorzugsweise ist das Halteelement 7, insbesondere gegen die Federkraft, verstellbar, um das Implantat 2 auszustoßen und/oder einzulegen.

Das Halteelement 7 kann durch den Vorschubmechanismus 5 verstellt werden, insbesondere bevor der Vorschubmechanismus 5 mit dem Implantat 2 in Eingriff kommt. Diese Verstellung des Halteelements 7, bevor der Vorschubmechanismus 5 mit dem Implantat 2 in Eingriff kommt, schützt das Implantat 2 vor Beschädigungen, da das Implantat 2 frei von dem Halteelement 7 verstellt werden kann. Eine solche vorauseilende Verstellung des Halteelements 7 kann auch unabhängig von der Federbeaufschlagung vorgesehen sein. Prinzipiell kann das Halteelement 7 eine Ruheposition aufweisen, in der das Halteelement 7 das Implantat 2 hält und durch den Vorschubmechanismus 5 aus der Ruheposition verstellt werden. Wie dargestellt kann das Halteelement 7 eine Anlaufschräge 21 aufweisen, die von dem Vorschubmechanismus 5 zur Verstellung des Halteelements 7 kontaktiert wird. Fig. 5 a) zeigt eine aktive Stellung des Halteelements 7 und Fig. 5b) zeigt, wie das Halteelement 7 ausgelenkt wird.

Zum Einlegen des Implantats 2 ist denkbar, dass das Halteelement 7 manuell verstellt wird oder erst nach dem Einlegen in eine aktive Stellung verbracht wird. Vorzugsweise kontaktiert das Halteelement 7 hier das Implantat 2 im vorderen Bereich. So wird verhindert, dass ein gebrochenes Implantat 2 nach vorne aus dem Injektor fällt. Eine Kontaktierung im vorderen Bereich kann aus dem gleichen Grund auch für das Halteelement 7 der Figuren 1 bis 4 vorgesehen sein. Das Haltelement 7 kann aus Kunststoff bestehen. Gegenüber beispielsweise einem Halteelement 7 aus Gummi kann so Abrieb vermieden werden. Das Halteelement 7 kann, wie dargestellt, schwenkbar sein.

Vorgeschlagen wird gemäß einer weiteren Lehre, der eigenständige Bedeutung zukommt, eine Injektoranordnung mit einem vorschlagsgemäßen Injektor 1 oder mit einem vorderen Abschnitt 10 mit einem Implantat 2 und einem Halteelement 7 für einen vorschlagsgemäßen Injektor 1.

Dabei ist vorgesehen, dass der Injektor 1 oder der vordere Abschnitt 10 steril verpackt ist. Die Injektoranordnung kann demnach eine, beispielsweise dampfsterilisierbare und dampfsterilisierte oder Ethylenoxid-sterilisierte oder mit Gammastrahlung sterilisierte, Verpackung aufweisen, in der der Injektor 1 oder der vordere Abschnitt 10 angeordnet ist. Im Operationssaal wird der Injektor 1 dann aus der Verpackung entnommen und verwendet und vorzugsweise anschließend entsorgt. Alternativ wird der vordere Abschnitt 10 aus der Verpackung entnommen und vorzugsweise auf einen Mehrweghauptabschnitt aufgesetzt.

Auf alle Ausführungen zu dem vorschlagsgemäßen Injektor 1 darf verwiesen werden.

Vorgeschlagen wird gemäß einer weiteren Lehre, der eigenständige Bedeutung zukommt, ein Verfahren zur Herstellung eines vorschlagsgemäßen Injektors 1.

Bei der Herstellung ist vorgesehen, dass das Implantat 2 und das Halteelement 7 in den Lagerabschnitt 4 eingebracht werden.

Auf alle Ausführungen zu dem vorschlagsgemäßen Injektor 1 und der vorschlagsgemäßen Injektoranordnung darf verwiesen werden.

Die Figuren 3 und 4 zeigen Varianten, wie der Injektor 1 hergestellt werden kann. Bei einer Variante, die in Fig. 3 a) und b) dargestellt ist, ist vorgesehen, dass das Halteelement 7 mit dem Implantat 2 verbunden wird und anschließend das Implantat 2 mit dem Halteelement 7 zusammen in den Lagerabschnitt 4 eingebracht wird. So kann beispielsweise eine stärkere Haftung zwischen Implantat 2 und Halteelement 7 als zwischen Halteelement 7 und Lagerabschnitt 4 erreicht werden.

Alternativ kann, wie in Fig. 3 c) und d) dargestellt, vorgesehen sein, dass erst das Halteelement 7 und anschließend das Implantat 2 in den Lagerabschnitt 4 eingebracht wird. Diese Variante kann zu einer stärkeren Haftung zwischen Lagerabschnitt 4 und Halteelement 7 führen. Weiter alternativ und in Fig. 4 a) dargestellt kann vorgesehen sein, dass erst das Implantat 2 und anschließend das Halteelement 7 in den Lagerabschnitt 4 eingebracht wird. So kann eine ähnlich starke Haftung erreicht werden. Letztlich hängt die Haftung jedoch beispielsweise auch von den Materialien ab, sodass die Reihenfolge des Einbringens nicht der einzige Einflussfaktor ist.

Es ist hier und vorzugsweise vorgesehen, dass das Halteelement 7 und/oder das Implantat 2 durch die weitere Öffnung 12 in den Lagerabschnitt 4 eingebracht wird.

Grundsätzlich ist möglich, dass das Halteelement 7 in einem flüssigen Zustand in den Injektor 1 eingebracht und/oder mit dem Implantat 2 verbunden wird.

Besonders in diesem Fall, jedoch auch allgemein, kann vorgesehen sein, dass das Halteelement 7 aktiv ausgehärtet, insbesondere UV-getrocknet, wird. Das Aushärten ist in den Figuren ebenfalls dargestellt und kann ebenfalls genutzt werden, um die Haftung nach Bedarf abzustimmen, abhängig davon, ob das Halteelement 7 mit ausgestoßen werden soll oder nicht. Bevorzugt ist es so, dass das Halteelement 7 durch die weitere Öffnung 12 aktiv ausgehärtet wird. Der Begriff "aktiv" bezieht sich dabei auf ein Aushärten, bei dem beispielsweise UV genutzt wird und nicht ein rein zeitbasiertes Aushärten. Genauso kann Vakuum verwendet werden, um das Implantat 2 auszuhärten, insbesondere zu trocknen. Auch ist ein Aushärten durch ein Aufheizen des vorderen Abschnitts 10 zu erreichen.

Weiter ist hier und vorzugsweise vorgesehen, dass der Injektor 1 oder der vordere Abschnitt 10 mit dem Implantat 2 sterilisiert wird.

## Patentansprüche

1. Injektor für ein Implantat (2), wobei das Implantat (2) in dem Injektor (1) gelagert ist, wobei der Injektor (1) eine Injektionsöffnung (3) zum Abgeben des Implantats (2) in ein Organ, insbesondere ein Auge, einen Lagerabschnitt (4), in dem das Implantat (2) gelagert ist, und einen Vorschubmechanismus (5) zum Ausstoßen des Implantats (2) von dem Lagerabschnitt (4) durch die Injektionsöffnung (3) aufweist,
**dadurch gekennzeichnet,**
**dass** das Implantat (2) mittels eines das Implantat (2) kontaktierenden Halteelements (7) im Lagerabschnitt (4) festgelegt ist.

2. Injektor nach Anspruch 1, **dadurch gekennzeichnet, dass** das Halteelement (7) biokompatibel ist, vorzugsweise, dass das Halteelement (7) beim Ausstoßen des Implantats (2) zumindest teilweise zerstört wird, und/oder, dass das Halteelement (7) beim Ausstoßen zumindest teilweise am Implantat (2) haften bleibt, und/oder, dass das Halteelement (7), insbesondere vollständig, aus einem Material besteht, aus dem das Implantat (2), zumindest teilweise, ebenfalls besteht.

3. Injektor nach Anspruch 1 oder 2, **dadurch gekennzeichnet, dass** das Halteelement (7), zumindest teilweise, aus einem Polymer besteht, vorzugsweise, dass das Polymer Polycaprolacton (PCL) oder Polylactide (PLA) oder Polyglycolsäure (PGA) oder Polylactid-co-Glycolid (PLGA) ist.

4. Injektor nach einem der vorhergehenden Ansprüche, **dadurch gekennzeichnet, dass** das Halteelement (7) am Implantat (2) und am Lagerabschnitt (4) haftet, vorzugsweise, dass das Halteelement (7) am Implantat (2) und/oder am Lagerabschnitt (4) stoffschlüssig haftet.

5. Injektor nach einem der vorhergehenden Ansprüche, **dadurch gekennzeichnet, dass** der Injektor (1) einen vorderen Abschnitt (10) aufweist, der insbesondere als hohle Nadelspitze (11) ausgestaltet ist, der den Lagerabschnitt (4) und die Injektionsöffnung (3) aufweist, dass der vordere Abschnitt (10) zusätzlich zur Injektionsöffnung (3) eine weitere Öffnung (12) aufweist, durch die das Halteelement (7) eingebracht wurde, vorzugsweise, dass die weitere Öffnung (12) eine hintere Öffnung (13) des vorderen Abschnitts (10) ist, durch die der Vorschubmechanismus (5) hindurch bewegt wird, oder, dass die weitere Öffnung (12) eine seitliche Öffnung (14) ist, die vorzugsweise nur dem Einbringen des Halteelements (7) dient und die weiter vorzugsweise von dem Halteelement (7) verschlossen wird.

6. Injektor nach Anspruch 5, **dadurch gekennzeichnet, dass** der vordere Abschnitt (10) als separates Bauteil ausgestaltet ist, vorzugsweise, dass der Injektor (1) einen Hauptabschnitt (15) aufweist, in dem der Vorschubmechanismus (5) verschiebbar geführt ist und an dem der vordere Abschnitt (10) befestigt ist, weiter vorzugsweise, dass der Hauptabschnitt (15) eine Transportsicherung (8) für den Vorschubmechanismus (5) aufweist.

7. Injektor nach einem der vorhergehenden Ansprüche, **dadurch gekennzeichnet, dass** das Halteelement (7) in einer hinteren, von der Injektionsöffnung (3) abgewandten, Hälfte, insbesondere einem hinteren Drittel, des Implantats (2) an dem Implantat (2) haftet, vorzugsweise, dass das Halteelement (7) an einem hinteren Ende (16) des Implantats (2) haftet.

8. Injektor nach einem der vorhergehenden Ansprüche, **dadurch gekennzeichnet, dass** der Vorschubmechanismus (5) ein Vorschubelement (17) und ein Antriebselement (18) aufweist, dass das Antriebselement (18) manuell von außerhalb des Injektors (1) verstellbar ist und das Vorschubelement (17), insbesondere linear, vorschiebt, und, dass das Vorschubelement (17) von hinten in Eingriff mit dem Implantat (2) kommt und das Implantat (2) vorschiebt.

9. Injektoranordnung mit einem Injektor (1) nach einem der vorhergehenden Ansprüche oder mit einem vorderen Abschnitt (10) mit einem Implantat (2) und einem Halteelement (7) für einen Injektor (1) nach einem der vorhergehenden Ansprüche
**dadurch gekennzeichnet,**
**dass** der Injektor (1) oder der vordere Abschnitt (10) steril verpackt ist.

10. Verfahren zur Herstellung eines Injektors (1) nach einem der Ansprüche 1 bis 8,
**dadurch gekennzeichnet,**
**dass** das Implantat (2) und das Halteelement (7) in den Lagerabschnitt (4) eingebracht werden.

11. Verfahren nach Anspruch 10, **dadurch gekennzeichnet, dass** das Halteelement (7) mit dem Implantat (2) verbunden wird und anschließend das Implantat (2) mit dem Halteelement (7) zusammen in den Lagerabschnitt (4) eingebracht wird, oder,
dass erst das Halteelement (7) und anschließend das Implantat (2) in den Lagerabschnitt (4) eingebracht wird, oder,
dass erst das Implantat (2) und anschließend das Halteelement (7) in den Lagerabschnitt (4) eingebracht wird.

12. Verfahren nach Anspruch 10 oder 11, **dadurch gekennzeichnet, dass** das Halteelement (7) und/oder das Implantat (2) durch die weitere Öffnung (12) in den Lagerabschnitt (4) eingebracht wird.

13. Verfahren nach einem der Ansprüche 10 bis 12, **dadurch gekennzeichnet, dass** das Halteelement (7) in einem flüssigen Zustand in den Injektor (1) eingebracht und/oder mit dem Implantat (2) verbunden wird.

14. Verfahren nach einem der Ansprüche 10 bis 13, **dadurch gekennzeichnet, dass** das Halteelement (7) aktiv ausgehärtet, insbesondere UV-getrocknet und/oder mittels Vakuum getrocknet, wird, weiter vorzugsweise, dass das Halteelement (7) durch die weitere Öffnung (12) aktiv ausgehärtet wird.

15. Verfahren nach einem der Ansprüche 10 bis 14, **dadurch gekennzeichnet, dass** der Injektor (1) oder der vordere Abschnitt (10) mit dem Implantat (2) sterilisiert wird.
